# EUROPEAN PATENT SPECIFICATION

(11) **EP 3 669 805 B1**
(45) Date of publication and mention of the grant of the patent: **08.05.2024**
(21) Application number: 19215798.0
(22) Date of filing: 12.12.2019
(51) Int. Cl.: A61B 18/12, A61B 18/14, A61B 18/00

(54) **TREATMENT DEVICE FOR ELECTRIC ARC TREATMENT**
BEHANDLUNGSVORRICHTUNG ZUR LICHTBOGENBEHANDLUNG
DISPOSITIF POUR TRAITEMENT À ARC ÉLECTRIQUE

(30) Priority: 21.12.2018 IT 201800020968
(43) Date of publication of application: 24.06.2020
(73) Proprietor: Acea Medica S.r.l., 20123 Milano (IT)
(72) Inventor: TASSI, Alessandro, 20143 Milano (IT)
(74) Representative: Lunati & Mazzoni S.r.L.

(56) References cited:
- US-A1- 2001 034 519
- US-A1- 2002 128 641
- US-A1- 2012 191 084
- US-A1- 2014 155 884

## Description

The present invention relates to a treatment device, for example an aesthetic one, for electric arc treatment, of the type specified in the preamble of Claim 1. There are currently known electric arc treatment devices, designed, for example, to perform aesthetic corrections of the skin.

These devices comprise an electric power supply that supplies an electrode placed at one end of the device.

The electrode, generally having a needle or ball end, is designed to be electrically charged by the electric power supply and to assume a high electrical potential. When the electrode is brought near the human skin and reaches a distance close to or less than a millimetre, the electrical potential of the electrode end is counteracted by the electrical potential of the skin itself. Said potential difference is sufficient to create an electric arc between the electrode end and the skin itself. The electric arc, in turn, locally develops high temperatures and, consequently, creates a micro sublimation or elimination of the skin itself at its connection with it. Said effect can be used on people treated for different purposes, in particular to aesthetically correct the skin. For example, this device has been shown to eliminate: eyelid ptosis, excess skin, skin hyperchromia, periumbilical striae, warts, moles, keloids, fibroids, xanthelasma, dyskeratosis, and scars.

Moreover, the same device does not create deep cuts in the dermis and other drawbacks typical of mechanical treatments, i.e. using traditional scalpels and the like.

It is also very precise and accurate.

US 2002/128641 discloses a treatment device according to the pre-amble of claim 1. US 2012/191084, US 2001/034519 and US 2014/155884 disclose further treatment devices.

The prior art described comprises some significant drawbacks.

In particular, although the skin and dermis are less stressed than they are with traditional methods, they are still stressed and thermally damaged.

Consequently, subjects who have undergone the aesthetic treatment need a few days' recovery.

Moreover, subjects who have undergone the aesthetic treatment, in particular very sensitive subjects, may find the latter irritating.

In this context, the technical task underlying the present invention is to devise an electric arc treatment device that is capable of substantially overcoming at least some of the above-mentioned drawbacks.

In the context of said technical task, an important purpose of the invention is to obtain an electric arc treatment device that causes less stress and damage to the skin and dermis of treated subjects, while maintaining the same results.

Another important purpose of the invention is to provide an electric arc treatment device that enables the treated subject to recover more quickly over time.

Not the least important purpose of the present invention is to obtain an electric arc treatment device that does not cause irritation even to the most sensitive subjects treated.

The technical task and the specified purposes are achieved by means of an electric arc treatment device as claimed in the appended claim 1.

Preferred embodiments are described in the dependent claims.

The characteristics and benefits of the invention will be clarified in the following detailed description of some preferred embodiments of the invention, with reference to the accompanying drawings, wherein:

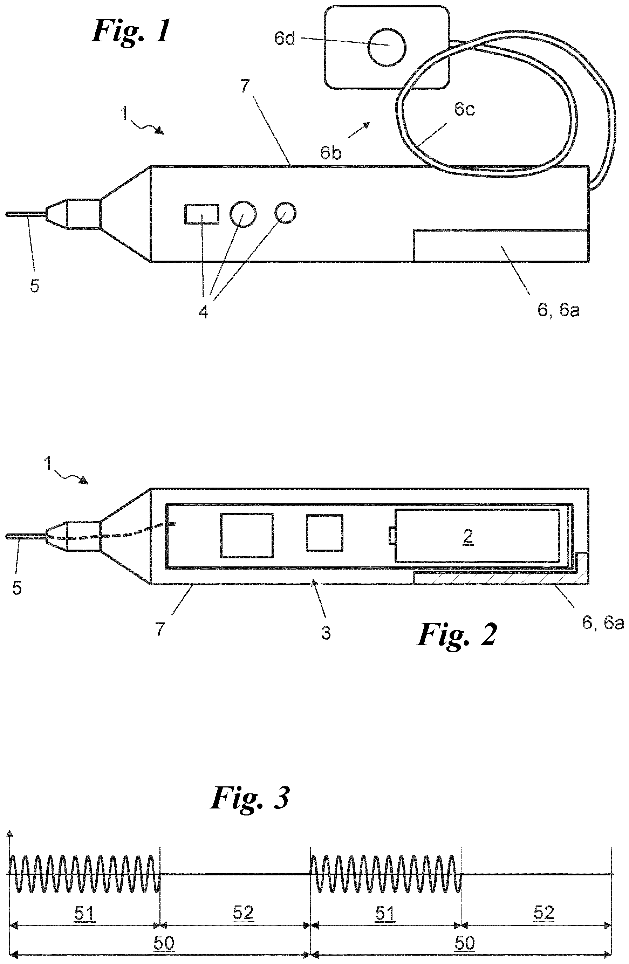

**Fig. 1** schematises the outer portion of the treatment device according to the invention;
**Fig. 2** schematises the interior of the treatment device according to the invention; and
**Fig. 3** schematises the operation of the treatment device according to the invention.

In this document, the measures, values, shapes, and geometric references (such as perpendicularity and parallelism), when associated with words like "about" or other similar terms such as "approximately" or "essentially", are to be understood as except for measurement errors or inaccuracies owing to production and/or manufacturing errors and, above all, except for a slight divergence from the value, measure, shape, or geometric reference with which it is associated. For example, these terms, if associated with a value, preferably indicate a divergence of not more than 10% of the value.

Furthermore, when used, terms such as "first", "second", "higher", "lower", "main" and "secondary" do not necessarily identify an order, a priority relationship, or a relative position, but can simply be used to more clearly distinguish between the different components.

The measurements and data contained in this text are to be considered, unless otherwise indicated, as carried out in ICAO International Standard Atmosphere (ISO 2533:1975).

With reference to the figures, the number **1** indicates, as a whole, the treatment device according to the invention.

The device is preferably used to aesthetically correct human skin. For example, the treatment device 1, is able to eliminate: eyelid ptosis, excess skin, skin hyperchromia, periumbilical striae, warts, moles, keloids, fibroids, xanthelasma, dyskeratosis, and scars.

The treatment device 1 preferably comprises, in summary, one or more of the following elements: power supply means **2**; control means **3**, of the treatment device 1; command means **4**, again, of the treatment device 1 and designed to enable a user to command a treatment electrode **5** arranged at least partially in contact with the external environment; and a reference electrode **6**, which can be electrically connected to a treated portion.

The power supply means 2 preferably consist of an electric battery, preferably a rechargeable and/or replaceable one. In order to recharge the power supply means, there is also, conveniently, a charging base (not shown in the figures) designed to be connected to the mains. The electric battery preferably has a voltage ranging between 8 and 20 V and a capacity ranging between 0.1 Ah and 1 Ah.

The control means 3 consist of an electrical circuit and processor capable of actuating said potential difference and the features specified below. The person skilled in the art can achieve these simply.

In detail, the control means 3 preferably comprise an inverter, from direct current to alternating current, and a transformer, which is designed to carry the potential difference from the voltage present at the ends of the electric battery, which constitutes the power supply means 2, to the voltage of the treatment electrode 5, with respect to the reference electrode 6, conveniently ranging between 2000 V (or preferably VAC) and 2200 V (or preferably VAC).

The control means 3 are, in addition, operationally connected to the command means 4. The latter preferably comprise one or more buttons and a screen, or the like, such as a touch screen. The command means 4 are therefore designed to allow a user to comfortably handle the treatment device 1.

Structurally, the treatment device 1 comprises an outer casing **7** preferably substantially cylindrical so that a user can grasp it. In addition, it is preferably largely made of electrically insulating material, preferably of polymer material.

The treatment electrode 5 is preferably arranged at one end of the outer casing 7. Conveniently, it is made of metal and preferably has a needle shape, more preferably a curved needle shape, so that a user can easily use it. It is also, preferably, easy to replace.

The treatment electrode 5 is electrically connected to the power supply means 2 and the control means 3 so that it can have a high electrical potential, for example, ranging between 2000 V and 2200 V.

On the other hand, in use the reference electrode 6 is preferably connected to the portion to be treated, in particular to the portion of human skin to be treated. It may comprise a first end **6a**, consisting of a conductive portion, preferably metallic, placed directly on the outer surface of the casing 7. In this way, the first end 6a is electrically connected to the portion to be treated through the skin of the operator's hand treating said portion. This hand, in fact, comes into contact with both the first end 6a and the portion to be treated.

The reference electrode 6 may also comprise outer electrodes **6b**, connected by means of a cable **6c** and a contact portion **6d** in contact with the device 1 and with the portion to be treated or near it. It is, preferably, easy to attach or detach from the outer casing 7. The contact portion 6d is, conveniently, an electrode of the type easily applicable to human skin.

In addition, the power supply means 2 preferably supply alternating current to the treatment electrode 5 by means of the control means 3 at frequencies ranging between 30 kHz and 50 kHz.

Consequently, in particular when the treatment electrode 5 is brought close to the portion to be treated, preferably at distances in the region of a millimetre, the electric potential difference creates an electric arc between the treated portion and the treatment electrode 5. This electric arc develops high temperatures and, for example, a sublimation of the treated skin in contact with the electric arc itself.

The command means 2, moreover, and always by means of the control means 3, are designed to activate and deactivate the supply of electric current to the treatment electrode 5 with a period **50** ranging between 0.5 ms and 20 ms and a duty cycle ranging between 0.02 and 0.8.

The period 50 therefore comprises an activation fraction **51**, during which said electric arc is created and remains, and a shut-down fraction **52**, during which said electric arc is not present. The ratio between the activation fraction 51 and the period 50 is called the duty cycle.

For example, the period 50 ranges between 8 ms and 12 ms and, preferably, the duty cycle ranges between 0.02 and 0.07.

As another example, the period 50 ranges between 3 ms and 7 ms and, preferably, the duty cycle ranges between 0.2 and 0.8, more preferably between 0.2 and 0.3 or between 0.6 and 0.7.

In another example, the period ranges between 1 ms and 3 ms and the duty cycle ranges between 0.4 and 0.6.

The operation of the treatment device 1 described above in structural terms, is as follows.

To carry out the treatment, the user carrying out the aesthetic treatment grasps the casing 7, inserts the treatment electrode 5, and, using the command means 5, sets the desired period and duty cycle, preferably choosing from a plurality of pre-set programs.

The user carrying out the treatment also places the reference electrode 6 in contact with the portion to be treated, in particular an area of the human skin, such as the face or another area. This contact is carried out by means of the user's hand and the first end 6a, or by the outer electrodes 6b.

The power supply means 2 and the control means 3 then charge the treatment electrode 5, preferably to the voltages indicated.

The user brings the treatment device 1 near to said portion to be treated, when the distance between the treatment electrode 5 and the same portion to be treated is sufficiently small, for example in the region of a millimetre, an electric arc is created between the portion to be treated, and in particular the skin, and the treatment electrode 5.

The electric arc sublimates, or in any case eliminates, the superficial portion of skin to be treated and eliminates the imperfections present.

Disclosed herein, but not claimed, is a new electric arc aesthetic treatment process by means of the treatment device 1 described above.

The treatment consists in supplying, by means of the power supply means 2 and/or the elements described above, to the treatment electrode 5, preferably alternated electric current and preferably with the frequencies previously indicated, so as to create an electric potential difference between the treatment electrode 5 and the reference electrode 6, which is preferably electrically connected to the treated portion or portion to be treated, so as to create an electric arc between the treated portion and the treatment electrode 5.

In said process, the supply of electric current to said treatment electrode 5 is conveniently activated and deactivated with a period 50 ranging between 0.5 ms and 20 ms and a duty cycle ranging between 0.02 and 0.8.

The treatment device 1 according to the invention achieves important advantages. In fact, following treatments administered with the same device 1, the skin and dermis of the user who has undergone treatment are less stressed than the skin and dermis of the user who has undergone treatment according to the traditional prior art, though the same benefits are obtained.

Consequently, subjects who have undergone aesthetic treatment administered with the device 1 need little time to recover.

Finally, even very sensitive subjects who have undergone the aesthetic treatment administered with the device 1 experience very little, basically non-existent, irritation.

The invention is susceptible of variations falling within the scope of the inventive concept as defined by the claims. In this context, the materials, shapes, and dimensions may be any materials, shapes, and dimensions.

## Claims

1. A treatment device (1) for electric arc treatment for aesthetically correct human skin comprising:
- power supply means (2),
- control means (3) for controlling said treatment device (1),
- command means (4) for commanding said treatment device (1), suitable to allow a user to command it,
- a treatment electrode (5), arranged at least partially in contact with the external environment,
- a reference electrode (6) electrically connectable to a treated portion,
- said power supply means (2) supplying said treatment electrode (5) with alternating electric current having frequencies ranging between 30 kHz and 50 kHz, so as to create an electric potential difference between said treatment electrode (5) and so that said electric potential difference creates an electric arc between said treated portion and said treatment electrode (5),
- said command means (4) being designed to activate and deactivate the supply of electric current to said treatment electrode (5) with a duty cycle ranging between 0.02 and 0.8, and
- **characterised in that** said command means (4) are also designed to activate and deactivate the supply of electric current to said treatment electrode (5) with a period ranging between 0.5 ms and 20 ms.

2. The treatment device (1) according to claim 1, wherein said period ranges between 8 ms and 12 ms.

3. The treatment device (1) according to claim 2, wherein said duty cycle ranges between 0.02 and 0.07.

4. The treatment device (1) according to claim 1, wherein said period ranges between 3 ms and 7 ms.

5. The treatment device (1) according to claim 4, wherein said duty cycle is selected from the ranges of 0.2 to 0.3 or 0.6 to 0.7.

6. The treatment device (1) according to claim 1, wherein said period ranges between 1 ms and 3 ms.

7. The treatment device (1) according to claim 6, wherein said duty cycle ranges between 0.4 and 0.6.

8. The treatment device (1) according to any one of the preceding claims, wherein said power supply means (2) are designed to create a maximum electrical potential ranging between 2000 V and 2200 V in said treatment electrode (5).

9. The treatment device (1) according to any one of the preceding claims, wherein said treated portion is a portion of human skin.

## Patentansprüche

1. Behandlungsvorrichtung (1) zur Lichtbogenbehandlung für ästhetisch korrekte menschliche Haut, umfassend:
- eine Stromversorgungsmittel (2),
- Steuermittel (3) zur Steuerung der Behandlungsvorrichtung (1),
- Befehlsmittel (4) zur Steuerung der Behandlungsvorrichtung (1), die geeignet sind, einem Benutzer die Steuerung zu ermöglichen,
- eine Behandlungselektrode (5), die zumindest teilweise in Kontakt mit der äußeren Umgebung steht,
- eine Referenzelektrode (6), die elektrisch mit einem behandelten Teil verbunden werden kann,
- wobei die Stromversorgungsmittel (2) die Behandlungselektrode (5) mit elektrischem Wechselstrom mit Frequenzen zwischen 30 kHz und 50 kHz versorgen, um eine elektrische Potentialdifferenz zwischen der Behandlungselektrode (5) zu erzeugen, so dass die elektrische Potentialdifferenz einen Lichtbogen zwischen dem behandelten Teil und der Behandlungselektrode (5) erzeugt,
- wobei die Befehlsmittel (4) so ausgelegt sind, dass sie die Zufuhr von elektrischem Strom zu der Behandlungselektrode (5) mit einem Tastverhältnis zwischen 0,02 und 0,8 aktivieren und deaktivieren, und
- **dadurch gekennzeichnet, dass** die Befehlsmittel (4) auch so ausgelegt sind, dass sie die Zufuhr von elektrischem Strom zu der Behandlungselektrode (5) mit einer Periode zwischen 0,5 ms und 20 ms aktivieren und deaktivieren.

2. Die Behandlungsvorrichtung (1) nach Anspruch 1, wobei die Periode zwischen 8 ms und 12 ms liegt.

3. Die Behandlungsvorrichtung (1) nach Anspruch 2, wobei das Tastverhältnis zwischen 0,02 und 0,07 liegt.

4. Die Behandlungsvorrichtung (1) nach Anspruch 1, wobei die Periode zwischen 3 ms und 7 ms liegt.

5. Die Behandlungsvorrichtung (1) nach Anspruch 4, wobei das Tastverhältnis aus den Bereichen 0,2 bis 0,3 oder 0,6 bis 0,7 ausgewählt ist.

6. Die Behandlungsvorrichtung (1) nach Anspruch 1, wobei die Periode zwischen 1 ms und 3 ms liegt.

7. Die Behandlungsvorrichtung (1) nach Anspruch 6, wobei das Tastverhältnis zwischen 0,4 und 0,6 liegt.

8. Die Behandlungsvorrichtung (1) nach einem der vorangehenden Ansprüche, wobei die Stromversorgungsmittel (2) so ausgelegt sind, dass sie in der Behandlungselektrode (5) ein maximales elektrisches Potential im Bereich zwischen 2000 V und 2200 V erzeugen.

9. Die Behandlungsvorrichtung (1) nach einem der vorangehenden Ansprüche, wobei der behandelte Teil ein Teil der menschlichen Haut ist.

## Revendications

1. Dispositif de traitement (1) pour le traitement à l'arc électrique de la peau humaine esthétiquement correcte, comprenant :
- des moyens d'alimentation (2) électrique,
- des moyens de contrôle (3) pour commander ledit dispositif de traitement (1),
- un moyen de commande (4) pour commander ledit dispositif de traitement (1), apte à permettre à un utilisateur de le commander,
- une électrode de traitement (5), disposée au moins partiellement en contact avec l'environnement extérieur,
- une électrode de référence (6) connectable électriquement à une partie traitée,
- ledit moyen d'alimentation (2) alimentant ladite électrode de traitement (5) en courant électrique alternatif de fréquence comprise entre 30 kHz et 50 kHz, de manière à créer une différence de potentiel électrique entre ladite électrode de traitement (5) et de manière à ce que ladite différence de potentiel électrique crée un arc électrique entre ladite partie traitée et ladite électrode de traitement (5),
- ledit moyen de commande (4) est conçu pour activer et désactiver l'alimentation en courant électrique de ladite électrode de traitement (5) avec un rapport cyclique compris entre 0,02 et 0,8, et
- **caractérisés en ce que** lesdits moyens de commande (4) sont également conçus pour activer et désactiver l'alimentation en courant électrique de ladite électrode de traitement (5) avec une période comprise entre 0,5 ms et 20 ms.

2. Dispositif de traitement (1) selon la revendication 1, dans lequel ladite période est comprise entre 8 ms et 12 ms.

3. Dispositif de traitement (1) selon la revendication 2, dans lequel ledit rapport cyclique est compris entre 0,02 et 0,07.

4. Le dispositif de traitement (1) selon la revendication 1, dans lequel ladite période est comprise entre 3 ms et 7 ms.

5. Dispositif de traitement (1) selon la revendication 4, dans lequel ledit rapport cyclique est choisi parmi les gammes de 0,2 à 0,3 ou de 0,6 à 0,7.

6. Le dispositif de traitement (1) selon la revendication 1, dans lequel ladite période est comprise entre 1 ms et 3 ms.

7. Le dispositif de traitement (1) selon la revendication 6, dans lequel ledit rapport cyclique est compris entre 0,4 et 0,6.

8. Dispositif de traitement (1) selon l'une quelconque des revendications précédentes, dans lequel lesdits moyens d'alimentation (2) sont conçus pour créer un potentiel électrique maximal compris entre 2000 V et 2200 V dans ladite électrode de traitement (5).

9. Le dispositif de traitement (1) selon l'une quelconque des revendications précédentes, dans lequel ladite portion traitée est une portion de peau humaine.
